# EUROPÄISCHE PATENTSCHRIFT

(11) **EP 1 404 242 B1**
(45) Veröffentlichungstag und Bekanntmachung des Hinweises auf die Patenterteilung: **22.02.2012**
(21) Anmeldenummer: 02762341.2
(22) Anmeldetag: 10.07.2002
(51) Int. Cl.: A61B 17/70

(54) **PEDIKELSCHRAUBENANORDNUNG**
PEDICULAR SCREW ARRANGEMENT
ENSEMBLE VIS PEDICULAIRE

(30) Priorität: 10.07.2001 EP 01116869
(43) Veröffentlichungstag der Anmeldung: 07.04.2004
(73) Patentinhaber: WALDEMAR LINK GmbH & Co. KG, 22339 Hamburg (DE)
(72) Erfinder: LINK, Helmut, D., 22397 Hamburg (DE)
(74) Vertreter: Glawe, Delfs, Moll
(86) Internationale Anmeldenummer: PCT/EP2002/007705
(87) Internationale Veröffentlichungsnummer: WO 2003/005917

(56) Entgegenhaltungen:
- FR-A- 2 735 351
- US-A- 5 527 312
- US-A- 5 645 599
- US-A- 5 733 284

## Beschreibung

Zur Stabilisierung, Ausrichtung oder Fusion von Wirbelkörpern verwendet man Apparate, die aus Pedikelschrauben und diese verbindenden Stangen bestehen. Die Pedikelschrauben werden zwischen dem processus costalis und dem processus articularis superior durch den Pedikel in den Wirbelkörper geschraubt. Der Pedikel ist äußerst kräftig und vermag die von den Pedikelschrauben übertragenen Kräfte aufzunehmen. Mitunter kommt es aufgrund der hohen Kräfte und insbesondere Biegemomente, die auf die Pedikelschrauben wirken, zu Schraubenbrüchen.

Dies kann man dadurch vermeiden (US-A-5 733 284), daß man die von den Stangen herrührenden Kräfte nicht über die Pedikelschrauben, sondern über Formstücke auf den Knochen überträgt, die dem Knochen angepaßt sind und Stützflächen aufweisen, die hakenförmig unter bzw. über Teile des Wirbelkörpers fassen. Die Pedikelschrauben dienen dann lediglich dazu, diese Formstücke am Knochen zu befestigen, ohne vertikale Kräfte übertragen zu müssen. Jedoch ist dieses System im Aufbau und operativ höchst aufwendig. Auch kann es nur dann wirksam sein, wenn die Anpassung an den Knochen weitgehend perfekt ist, was in der Praxis kaum zu erreichen ist.

Ferner sind Formstücke zum Verbinden längs verlaufender Stangen mit einer Reihe von Wirbelkörpern bekannt (US-A-4 697 582, US-A-6 136 002, US-A-4 697 582, US-A-6 136 002, US-A-4 289 123), die nicht speziell für Pedikelschrauben vorgesehen sind und auch keine über bzw. unter einen Knochen greifenden Stützflächen aufweisen.

Die Erfindung geht daher von dem eingangs genannten Stand der Technik aus, bei dem vorgesehen ist, daß die parallel zur Wirbelsäulenrichtung wirkenden Kräfte hauptsächlich über die Pedikelschrauben übertragen werden. Es liegt ihr die Aufgabe zugrunde, die Kraftübertragung im Bereich der Schrauben zu verbessern. Sie erreicht dies durch die Merkmale des Anspruchs 1 sowie vorzugsweise die Merkmale der Unteransprüche.

Gemäß der Erfindung umfaßt die Pedikelschraubenanordnung eine Pedikelschraubenstütze. Diese weist zum einen eine dem Schaft der Pedikelschraube angepaßte Bohrung auf, die den Schraubenschaft eng umschließt und abstützt. Zum anderen umfaßt sie ein Formstück, das mindestens eine zur ober- und/oder unterseitigen Anlage am Knochen ausgebildete Stützfläche aufweist. Damit diese einen Teil der fraglichen Kräfte von der Schraube auf den Knochen übertragen kann, verläuft sie möglichst weitgehend parallel zur Schraubenrichtung. Das heißt, daß sie im Sagittalschnitt mit der Richtung der Durchgangsöffnung (die auch die Schraubenrichtung ist) einen hinreichend kleinen Winkel einschließen muß, der vorzugsweise geringer als 40° ist. Vorzugsweise enthält sie einen Anteil, in welchem dieser Winkel kleiner als 20°, weiter vorzugsweise kleiner als 10° ist. Die Abstützung findet vorzugsweise oberhalb bzw. unterhalb der Schraubeneintrittsstelle an der Sattelfläche zwischen dem processus articularis superior und dem processus costalis statt. Andere geeignete Orte zur Abstützung sind die Unterseite bzw. Oberseite des processus costalis oder die Unterseite des processus articularis superior. Auch eine oberseitige Abstützung am processus articularis superior ist denkbar.

Die Bohrung in der Pedikelschraubenstütze soll so lang sein, daß sie im wesentlichen Richtungsgleichheit zwischen der Schraube und der Bohrung gewährleistet. Erfindungsgemäß ist die Bohrung von einem Rohrteil gebildet, das starr mit dem die Stützflächen bildenden Formstück verbunden sein kann. Die Stützfläche ist von der konkaven Seite des Formstücks gebildet, und der Rohrteil ragt von der konvexen Seite des Formstücks vor, um den Schraubenschaft an dieser Stelle abzustützen und gegenüber Biegebeanspruchung zu verstärken. Der Hebelarm des auf den Schraubenschaft wirkenden Biegemoments wird auf diese Weise verkürzt und die Beanspruchung der Schraube herabgesetzt.

Statt dessen oder außerdem kann vorgesehen sein, daß ein an der Bildung der Bohrung beteiligter Rohrteil zur anderen Seite von dem die Stützfläche(n) bildenden Formstück vorragt. Da das Formstück normalerweise im Bereich der Schraubeneintrittsstelle an der Knochenoberfläche anliegt, erstreckt sich dieser Rohrteil in den Knochen hinein. Für seine Aufnahme wird der Knochen etwas stärker formschlüssig aufgefräst oder aufgebohrt, als dies bislang zur Aufnahme lediglich des Schraubenschafts üblich ist. Der im Vergleich mit dem Schraubenschaft größere Durchmesser des Rohrteils ergibt eine entsprechend größere Kraftübertragungsfläche zum Knochen hin. Zusätzlich zu den ober- bzw. unterseitig sich an den Knochen anlegenden Stützflächen wird dadurch die Gefahr einer den Knochen verformenden Verlagerung der Schraube unter hoher Belastung verringert. Außerdem wird die Strecke vergrößert, innerhalb welcher der Schraubenschaft eine seine Beanspruchung senkende Abstützung vorfindet. Zweckmäßigerweise ist das knochenseitig vorragende, in den Knochen hineinragende Rohrstück außenseits mindestens teilweise konisch.

Es sollte nach einem weiteren Merkmal der Erfindung so lang sein, daß es mindestens bis zur Stützfläche vorragt. Dies hat den Vorteil, daß die Kraftübertragung vom Schraubenschaft auf die Pedikelschraubenstütze und von deren Stützflächen auf den Knochen nicht zu einem Biegemoment führt, das dazu neigen könnte, die Position der Pedikelschraubenstütze zu verlagern. Vorteilhafterweise ist dieser Rohrteil noch länger, nämlich mit einer im Knochen unterzubringenden Gesamtlänge in der Größenordnung von 1 bis 1,5 cm.

Wenn es sich um ein Dauerimplantat handelt, kann es zweckmä-βig sein, die Außenseite des Rohrteils so zu gestalten, daß eine innige Verbindung mit dem Knochen begünstigt wird. Dies kann zum Beispiel durch eine geeignete, porige Oberflächenform oder wachstumsfördernde Beschichtung geschehen.

Der Sitz des die Stützfläche(n) bildenden Formstücks am Knochen kann gegebenenfalls durch zum bzw. in den Knochen vorragende Zähne oder Schrauben verbessert werden.

Es ist im allgemeinen nicht erforderlich, daß die Stützflächen nach der Implantation unmittelbar und spaltfrei an der Knochenoberfläche anliegen. Vielmehr genügt es in den meisten Fällen, daß sie einen hinreichend geringen Abstand von der Knochenoberfläche haben, um ihre Stützwirkung dann zu entfalten, wenn sich der Schraubenschaft im Knochen unter der Krafteinwirkung verlagern sollte.

Normalerweise ist die Pedikelschraubenstütze nur je einer Pedikelschraube zugeordnet. Dies gestattet es, sie verhältnismäßig klein auszubilden, was die operative Verwendung erleichtert und die Möglichkeit ungenügender Anpassung an die Knochenform verringert; entsprechend klein kann die Zahl der bereit zu haltenden Größenstufen sein.

Die Erfindung wird im folgenden näher unter Bezugnahme auf die Zeichnung erläutert. Darin zeigen:
- Fig. 1: einen Sagittalschnitt durch die Pedikelschraubeneintrittsstelle einer ersten Ausführungsform,
- Fig. 2: eine Dorsalansicht in Richtung der Pedikelschraubenachse,
- Fig. 3: eine Ansicht dieser Region von oben;
- Fig. 4-6: zeigen entsprechende Darstellungen einer zweiten,
- Fig. 7-9: entsprechende Darstellungen einer dritten Ausführungsform und
- Fig. 10-12: vergrößerte Darstellungen von Ausführungsvarianten der Pedikelschaubenstütze, wobei Fig. 11 eine nicht erfindungsgemäße Ausführungsform zeigt.

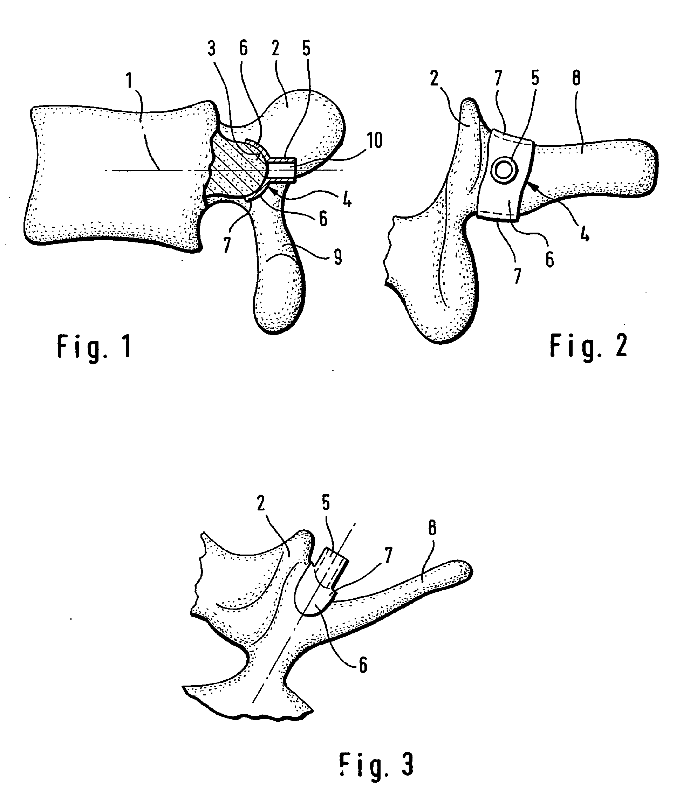

In Fig. 1 ist eine Pedikelschraube durch ihre strichpunktierte Achse 1 dargestellt. Sie tritt zwischen dem processus articularis superior 2 und dem vor der Zeichenebene liegenden processus costalis in die Oberfläche des Sattels 3 zwischen diesen beiden Fortsätzen in den Knochen ein.

Die Pedikelschraubenstütze 4 weist einen Rohrteil 5 zum Abstützen des Schraubenschafts auf. Eine hinreichend enge Passung sorgt dafür, daß die Stütze 4 an etwaigen Bewegungen des Schraubenschafts teilnehmen muß oder - umgekehrt ausgedrückt -, daß der Schraubenschaft sich an ihr abstützt, wenn sie dessen Bewegungstendenzen nicht folgen kann.

Die Stütze 4 umfaßt ein starr mit dem Rohrteil 5 verbundenes Formstück, das aus passend zu der Sattelfläche 3 gebogenen Flügeln 6 besteht, auf, die sich an die Satteloberfläche 3 weitgehend anlegen. Sie umfassen diese so weit, daß ihre Endbereiche 7 sich einer Richtung parallel zur Richtung 1 des Schraubenschafts bis auf weniger als 40°, vorzugsweise weniger als 20°, vorzugsweise weniger als 10°, annähern. Diese Endbereiche bilden die Stützflächen 7. Da sie im wesentlichen parallel zur Schraubenrichtung 1 verlaufen, sind sie in der Lage, die Schraube bei der Übertragung von Querkräften auf den Knochen zu unterstützen. Die Flügel 6 können mit Zähnen oder Schrauben versehen sein, die in die Sattelfläche 3 eindringen, um der Pedikelschraubenstütze 4 zusätzliche Stabilität am Knochen zu verleihen. Besonders deutlich sieht man in Fig. 1, wie die Flügel sich mit ihren Stützflächen 7 über und unter die Sattelfläche legen, die zwischen dem processus articularis superior 2 und dem processus costalis 8 liegt. Dasselbe geht aus der Draufsicht in Fig. 3 bezüglich des Flügels 6 hervor.

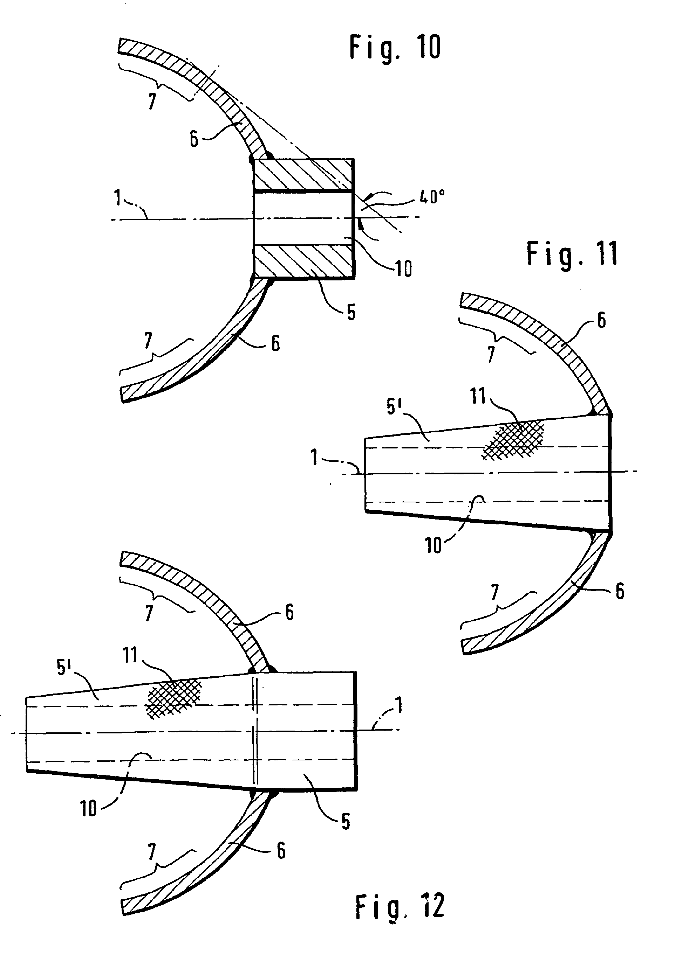

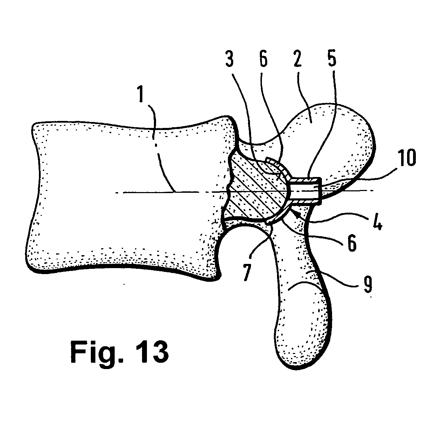

Einzelheiten der Pedikelschraubenstütze 4 ergeben sich aus der vergrößerten Darstellung in Fig. 10. Zur Aufnahme der Pedikelschraube weist sie einen Rohrteil 5 auf, der auf der dem Knochen abgewandten, konvexen Seite des Formstücks 6 vorragt.

Die Bohrung 10 innerhalb des Rohrteils 5 ist zylindrisch und entspricht mit geringem Spiel dem Außendurchmesser der nicht dargestellten Pedikelschraube. Die Pedikelschraubenstütze 4 ist daher stets richtungsgleich mit der Pedikelschraube ausgerichtet mit gemeinsamer Mittellinie 1.

Der Winkel, den die Flügel 6 an jeder Stelle im Sagittalschnitt mit der Richtung 1 einschließen, vermindert sich zu den Flügelenden hin, bis er den Betrag von 40° unterschreitet. Gemäß der Definition des Anspruchs 1 beginnen dann die Stützflächen 7.

Der Winkel von 40° ist willkürlich festgesetzt. Zwar versteht es sich, daß die Fähigkeit der Stützflächen zur Übertragung von quer zur Pedikelschraube verlaufenden Kräften um so besser ist, je geringer der Winkel ist, den sie mit der Achsrichtung 1 einschließen, so daß es unzweckmäßig erscheint, zu große Winkel zu wählen. Jedoch will die Erfindung nicht ausschließen, daß der erfindungsgemäße Stützeffekt je nach dem technischen Gesamtbild auch dann zustande kommen kann, wenn der Winkel größer als 40° ist. Dieses Anspruchsmerkmal ist demnach unter Berücksichtigung des erreichten Effekts auszulegen.

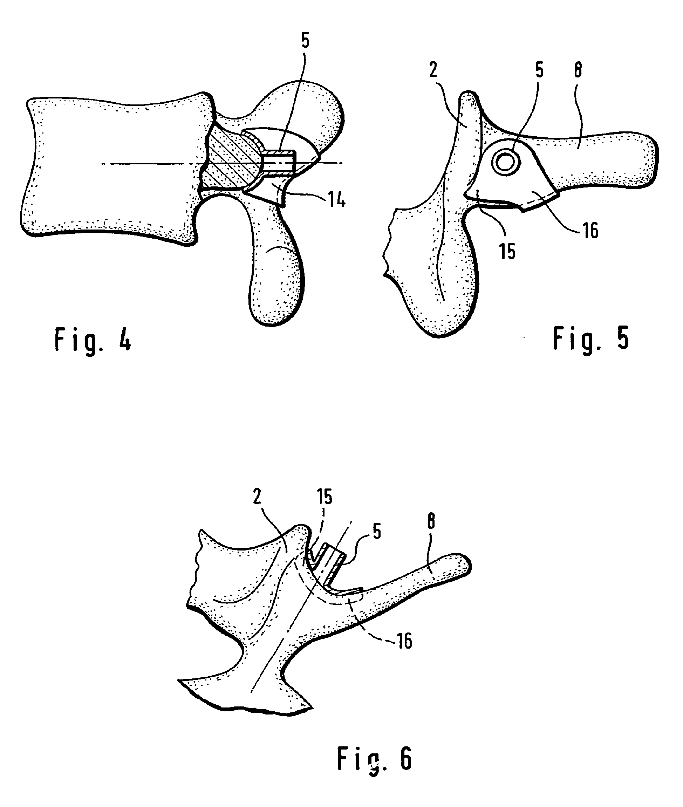

Das zweite Ausführungsbeispiel gemäß Fig. 4 bis 6 zeigt, daß der Rohrteil 5, der den Schraubenschaft eng umgeben soll, mit einem Blech-Formstück 14 starr verbunden ist, das mit einem ersten Flügel 15 unter den processus articularis superior und mit einem zweiten Flügel 16 unter den processus costalis greift. Kräfte, die zur Anhebung des Schraubenschafts tendieren, werden dadurch sicher auf die genannten Knochenflächen übertragen.

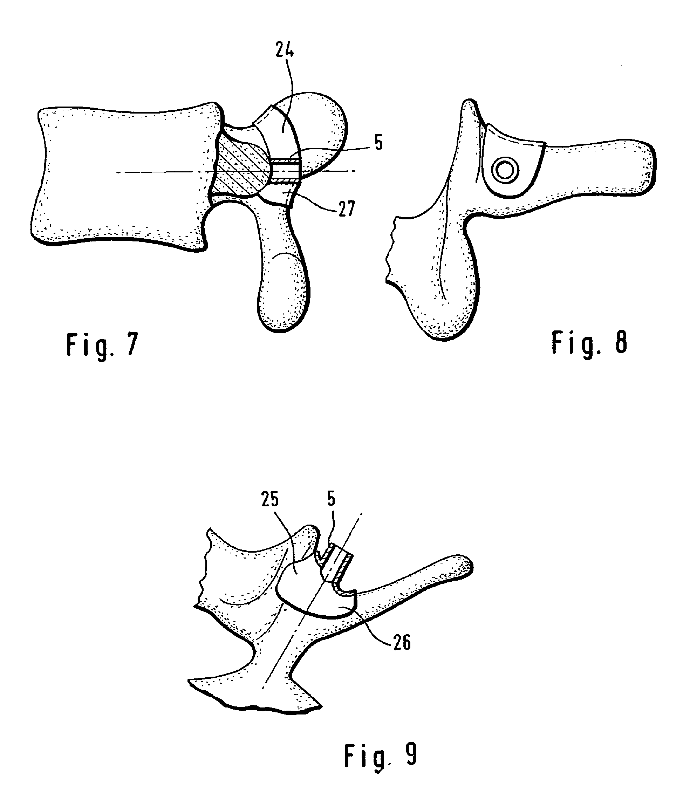

Das Ausführungsbeispiel gemäß Fig. 7 bis 9 zeigt Entsprechendes für eine oberseitige Abstützung. Der den Schraubenschaft aufnehmende Rohrteil 5 ist starr mit einem Blech-Formstück 24 verbunden, das mit einem ersten Flügel 25 über den processus articularis superior und mit einem zweiten Flügel 26 über den processus costalis greift. Es kann ein weiterer Flügel 27 vorhanden sein, der unter den processus articularis superior und/oder unter den processus costalis greift. Die Ausführungsformen gemäß Fig. 4 bis 6 einerseits und Fig. 7 bis 9 andererseits können somit in einem Formstück vereinigt sein.

Die Ausführungsbeispiele gemäß Fig. 1 bis 10 zeigen Rohrteil 5 auf der dem Knochen abgewendeten, konvexen Seite des Formstücks 6. Bei der nicht erfindungsgemäßen Ausführung gemäß Fig. 11 ist der Rohrteil 5' auf der anderen, dem Knochen zugewendeten, konkaven Seite des Formstücks 6 angeordnet. Sein Außendurchmesser verjüngt sich konisch zu seinem Ende hin. Seine Länge ist so gewählt, daß es sich (gemessen parallel zur Mittellinie 1) mindestens bis zum Beginn der Stützfläche 7 erstreckt, vorzugsweise aber darüber hinaus. Seine Gesamtlänge liegt in der Größenordnung von 1 bis 1,5 cm. Für die Aufnahme des Rohrteils 5' wird im Knochen eine formentsprechende Bohrung eingearbeitet. Diese kann im Verhältnis zur Außenform des Rohrteils 5' knapp ausgebildet sein, um nach dem Einsetzen oder Einschlagen des Rohrteils 5' einen festen, kraftaufnahmefähigen Sitz zu bilden.

Die Rohrteilausführungen gemäß Fig. 10 und Fig. 11 können miteinander kombiniert sein, wie es Fig. 12 zeigt.

Wenn die erfindungsgemäße Vorrichtung als Dauerimplantat vorgesehen ist, ist eine innige Verbindung des im Knochen sitzenden Rohrteils 5' mit dem umgebenden Knochen erwünscht. Zu diesem Zweck kann die Oberfläche dieses Rohrteils 5' - wie bei 11 schematisch angedeutet - mit einer Oberflächengestaltung versehen sein, die den innigen Knochenverbund fördert. Beispielsweise kann es sich um Aufrauhungen oder poröse Beschichtungen oder knochenwachstumsanregende Beschichtungen, beispielsweise aus Hydroxylapatit, handeln.

Die Knochenformen in dem betreffenden Bereich sind äußerst vielgestaltig. Zweckmäßig ist es deshalb, eine mehr oder weniger große Anzahl von Formstücken je nach Anwendungserfordernis bereitzustellen. Es besteht auch die Möglichkeit, die Pedikelschraubenstütze aus mehreren Teilen, die zur Abstützung an unterschiedlicher Stelle geeignet sind, mittels entsprechender Verbindungseinrichtungen an Ort und Stelle starr zusammenzufügen oder wenigstens mit dem Schraubenschaft zu verbinden.

Auch eine Abstützung an dem processus articularis inferior, der in Fig. 1 mit der Bezugsziffer 9 bezeichnet ist, kann im Rahmen der Erfindung stattfinden.

## Patentansprüche

1. Pedikelschraubenanordnung mit einer Pedikelschraubenstütze (4, 14, 24), die eine dem Schaft der Pedikelschraube (1) angepaßte Bohrung (10) und mindestens eine zur ober- und/oder unterseitigen Anlage an dem processus costalis (8) und/oder dem processus articularis superior (2) und/oder an der zwischen diesen befindlichen Sattelfläche (3) und/oder an den processus articularis inferior ausgebildete Stützfläche (7) aufweist, **dadurch gekennzeichnet, daß** die Bohrung (10) von einem Rohrteil (5, 5') gebildet ist und daß die Stützfläche (7) von der konkaven Seite eines Formstücks (6) gebildet ist und der Rohrteil (5) von der konvexen Seite des Formstücks (6) vorragt.

2. Pedikelschraubenanordnung nach Anspruch 1, **dadurch gekennzeichnet, daß** der Rohrteil (5') auf der konkaven Seite des Formstücks (6) von diesem vorragt.

3. Pedikelschraubenanordnung nach Anspruch 2, **dadurch gekennzeichnet, daß** der von der konkaven Seite des Formstücks (6) vorragende Rohrteil (5') außenseits mindestens teilweise konisch ist.

4. Pedikelschraubenanordnung nach Anspruch 2 oder 3, **dadurch gekennzeichnet, daß** der Rohrteil (5') auf der konkaven Seite mindestens bis zur Stützfläche (7) vorragt.

5. Pedikelschraubenanordngung nach einem der Ansprüche 2 bis 4, **dadurch gekennzeichnet, daß** die Außenseite des von der konkaven Seite des Formstücks (6) vorragenden Rohrteils (5') eine die Verbindung mit dem Knochen begünstigende Gestaltung (11) aufweist.

6. Pedikelschraubenanordnung nach einem der Ansprüche 1 bis 5, **dadurch gekennzeichnet, daß** die Pedikelschraubenstütze mit Einrichtungen zur Verbesserung ihres Sitzes an der Knochenoberfläche, insb. Zähnen, Schrauben oder einer bioaktiven Beschichtung, ausgerüstet ist.

7. Pedikelschraubenanordnung nach einem der Ansprüche 1 bis 6, **dadurch gekennzeichnet, daß** die Pedikelschraubenstütze (4, 14, 24) nur je einer Pedikelschraube zugeordnet ist.

## Claims

1. Pedicular screw arrangement with a pedicular screw support (4, 14, 24) which has a bore hole (10) adapted to the shank of the pedicular screw (1), and at least one supporting surface (7) which is designed for bearing from above and/or below on the costal process (8) and/or the superior articular process (2) and/or on the saddle surface (3) located between these and/or on the inferior articular process, **characterized in that** the bore hole (10) is formed by a tube part (5, 5') and **in that** the supporting surface (7) is formed by the concave side of a shaped piece (6), and the tube part (5) protrudes from the convex side of the shaped piece (6).

2. Pedicular screw arrangement as claimed in Claim 1, **characterized in that** the tube part (5') protrudes from the concave side of the shaped piece (6).

3. Pedicular screw arrangement as claimed in Claim 2, **characterized in that** the tube part (5') protruding from the concave side of the shaped piece (6) is at least partially conical on the outside.

4. Pedicular screw arrangement as claimed in Claim 2 or 3, **characterized in that** the tube part (5') protrudes from the concave side at least as far as the supporting surface (7).

5. Pedicular screw arrangement as claimed in one of Claims 2 through 4, **characterized in that** the outside of the tube part (5') protruding from the concave side of the shaped piece (6) has a configuration (11) favoring connection to the bone.

6. Pedicular screw arrangement as claimed in one of Claims 1 through 5, **characterized in that** the pedicular screw support is equipped with devices for improving its fit on the bone surface, in particular with teeth, screws, or a bioactive coating.

7. Pedicular screw arrangement as claimed in one of Claims 1 through 6, **characterized in that** the pedicular screw support (4, 14, 24) is assigned in each case to only one pedicular screw.

## Revendications

1. Ensemble de vis pédiculaire avec un appui de vis pédiculaire (4, 14, 24), qui présente un alésage (10) adapté à la tige de la vis pédiculaire (1) et au moins une face d'appui (7) réalisée en vue de l'application côté supérieur et/ou côté inférieur sur le processus costalis (8) et/ou le processus articularis superior (2) et/ou sur la face de selle (3) se trouvant entre ceux-ci et/ou sur le processus articularis inferior, **caractérisé en ce que** l'alésage (10) est formé par une partie tubulaire (5, 5') et **en ce que** la face d'appui (7) est formée par le côté concave d'une pièce moulée (6) et la partie tubulaire (5) est saillante sur le côté convexe de la pièce moulée (6).

2. Ensemble de vis pédiculaire selon la revendication 1, **caractérisé en ce que** la partie tubulaire (5') est saillante sur la pièce moulée (6) sur le côté concave de celle-ci.

3. Ensemble de vis pédiculaire selon la revendication 2, **caractérisé en ce que** la partie tubulaire (5') saillante sur le côté concave de la pièce moulée (6) est au moins partiellement conique sur le côté extérieur.

4. Ensemble de vis pédiculaire selon la revendication 2 ou 3, **caractérisé en ce que** la partie tubulaire (5') est saillante sur le côté concave au moins jusqu'à la face d'appui (7).

5. Ensemble de vis pédiculaire selon l'une quelconque des revendications 2 à 4, **caractérisé en ce que** le côté extérieur de la partie tubulaire (5') saillante sur le côté concave de la pièce moulée (6) présente une configuration (11) favorisant la liaison avec l'os.

6. Ensemble de vis pédiculaire selon l'une quelconque des revendications 1 à 5, **caractérisé en ce que** l'appui de vis pédiculaire est doté de dispositifs destinés à améliorer son placement sur la surface de l'os, en particulier des dents, des vis ou un revêtement bioactif.

7. Ensemble de vis pédiculaire selon l'une quelconque des revendications 1 à 6, **caractérisé en ce que** l'appui de vis pédiculaire (4, 14, 24) n'est associé chaque fois qu'à une seule vis pédiculaire.
